# EUROPEAN PATENT APPLICATION

(11) **EP 2 389 931 A1**
(43) Date of publication of application: **30.11.2011**
(21) Application number: 10163511.8
(22) Date of filing: 21.05.2010
(51) Int. Cl.: A61K 31/167, A61K 31/385, A61K 47/00, A61K 47/26, A61K 9/00

(54) **Pharmaceutical aqueous compositions comprising lipoic acid as an antioxidant**

(71) Applicant: Raw Materials Internatinal LLC, 6901 Lugano (CH)
(72) Inventor: De Tommaso, Vincenzo, 20052, Monza (MI) (IT)
(74) Representative: Serravalle, Marco

(57) **Abstract**

The present invention is directed to a stable injectable pharmaceutical aqueous solution comprising a pharmaceutical compound and lipoic acid as an antioxidant. A non-limiting list of pharmaceutical compounds useful in the present invention are: paracetamol, fructose 1,6 diphosphate, promethazine, amikacin, dobutamine and diclofenac. The solutions according to the invention preferably comprise a non-ionic surfactant which helps solubilization of lipoic acis. A preferred non-ionic surfactant is glychocolic acid.

## Description

The present invention is directed to a stable injectable pharmaceutical aqueous solution comprising a pharmaceutical compound and lipoic acid as an antioxidant. Many pharmaceutical compounds are commercially available as stable injectable aqueous solutions. These pharmaceutical solutions usually comprise an antioxidant to guarantee long term stability of the solution.

In the vast majority of the cases, injectable solutions are stabilized by addition of sodium metabisulfite. However, it is known that this compound can cause anaphylactic shock. This is why the use of this compound in pharmaceutical compositions is subjected to limitations.

Consequently there is the need for safe and effective antioxidants for the stabilization of pharmaceutical injectable aqueous solutions.

EP 0 858 329 discloses a paracetamol aqueous solution comprising water, a polyol or a water soluble alkanol and a free radical scavenger or an anti-radical agent. The solution, to be stable, needs to be deoxygenated by bubbling an inert gas. Preferred free radical scavengers are derivatives of ascorbic acid, organic compounds which bear at least one thiol function and polyols.

Lipoic acid is a well known natural compound. In 1966 its use for the treatment of diabetes was approved in Germany and in the 1980s it became available as a nutritional supplement for its known properties as an in vivo antioxidant. However, we are not aware of any use of lipoic acid as an antioxidant for aqueous pharmaceutical compositions.

We have surprisingly found that the use of lipoic acid as an antioxidant, produces injectable pharmaceutical solutions that are stable. Pharmaceutical compounds that can be stabilized by the use of lipoic acid are not particularly limited and can for example belong to the category of analgesic, antipyretic, anti-inflammatory, anti-staminic, anti-emetic, antibiotic, corticosteroid, anti-depressive, anti-epilepsy and inotropic compounds.

A non-limiting list of pharmaceutical compounds useful in the present invention are: paracetamol, fructose 1,6 diphosphate, promethazine, amikacin, dobutamine and diclofenac.

The solution according to the present invention preferably comprises a non-ionic surfactant, whose presence is useful in dissolving lipoic acid. The non-ionic surfactant used in the composition can be any non-ionic surfactant suitable for pharmaceutical compositions. Examples of preferred non-ionic surfactants are glychocolic acid, glycofurol, polyethylenglycol 200, polyethylene-glycol 300 and polyethylenglycol 400; the most preferred non-ionic surfactant is glychocolic acid. The non-ionic surfactant is present in a weight ratio with lipoic acid comprised between 1:10 and 10:1, preferably from 5:1 to 1:5.

In a preferred embodiment the invention is directed to the paracetamol solutions comprising lipoic acid as an antioxidant. More particularly, the invention is directed to a pharmaceutical composition comprising water, paracetamol, an amino sugar, a non-ionic surfactant and lipoic acid as an antioxidant. It is known that paracetamol, when stored in aqueous solutions tends to degrade with formation of p-aminophenol. It has been surprisingly found that lipoic acid can stabilize paracetamol in aqueous solution in a more effective way than thiol compounds. In fact, the aqueous solutions according to the invention do not require bubbling with an inert gas and are stable even in the presence of dissolved oxygen.

The concentration of lipoic acid to be used in paracetamol solutions according to the invention is preferably comprised between 0.05 g/L and 2 g/L, more preferably 0.1 and 1 g/L.

The paracetamol solutions according to the invention are solutions ready for use, i.e. solution wherein paracetamol is present in a concentration comprised between 5 and 20 g/L. The solution can also comprise another active ingredient which is commonly used in combination with paracetamol. For example, the solution can also comprise a compound selected from the group consisting of an analgesic, an anti-inflammatory compound, an antiemetic, a corticosteroid, an antidepressive and an anti-epilepsy compound.

The aminosugar used in this embodiment of the present invention is important to help solubilization of paracetamol. Preferred examples of aminosugars useful in the present invention are: glucamine, glucosamine, chondrosamine and their N-alkyl derivatives such as N-methylglucamine. The weight ratio between aminosugar and paracetamol preferably varies between 0.01:1 and 0.5:1. If the amount of aminosugar is too low, the solubility of paracetamol will be lower than desired.

It has been surprisingly found that aqueous solutions comprising paracetamol and lipoic acid are stable and do not require bubbling with an inert gas to remain stable for a long period. Thus, in a preferred embodiment of the invention, the paracetamol solution is not degassed to remove oxygen.

In a preferred embodiment, the paracetamol aqueous solutions according to the invention are prepared by a process comprising: dissolving lipoic acid and aminosugar in water; when lipoic acid is solubilized, a non-ionic surfactant is added, preferably under stirring and the pH is brought to a value in the range from 9 to 10, preferably about 9.5, by adding a base, preferably NaOH; when also the surfactant is dissolved, paracetamol is added and the dispersion kept under stirring until complete solubilization is obtained; then, pH is brought to a value close to neutral, preferably from 5 to 8, more preferably from 5.5 to 6.5, most preferably about 6, by adding an acid, preferably phosphoric acid; the solution is brought to the final volume by addition of water and the pH of the final solution controlled and corrected to the previous value by adding the acid or the base previously used.

The obtained solution is then treated according to standard methods used in the pharmaceutical field to prepare sterile vials. For example, the solution can be filtered on a 0.22 µm filter and bottled sterilely.

In another preferred embodiment of the invention lipoic acid is used to stabilize aqueous solutions of fructose 1,6 sodium diphosphate.

Fructose 1,6 sodium diphosphate is highly soluble in water, thus it does not require addition of any cosolvent. These solutions are based on water, fructose 1,6 diphosphate and lipoic acid. The amount of lipoic acid required to achieve stabilization of the solution is comprised between 0.1 g/L and 5 g/l, preferably between 0.2 g/L and 2 g/L. The amount of fructose 1,6 diphosphate in the solution, due to its solubility, can vary in a broad range. A preferred concentration range is from 10 g/L to 200 g/L, more preferably between 50 and 150 g/L.

The solution is preferably prepared by adding the amount of lipoic acid and glychocolic acid in about 70 % of the final volume of water. Lipoic acid is solubilized by adding a base, preferably sodium hydroxide. Then fructose 1,6 sodium diphosphate is added under stirring. The pH is maintained in the range from 4.5 and 5.5, preferably from 4.8 to 5.2. The solution is brought to the final volume with water.

The obtained solution is then treated according to standard methods used in the pharmaceutical field to prepare sterile vials. For example, the solution can be filtered on a 0.22 µm filter and bottled sterilely.

Another preferred embodiment of the invention concerns the preparation of aqueous solutions of dobutamine chlorohydrate stabilized by lipoic acid. Dobutamine chlorohydrate is commonly sold in 20 ml vials containing 250 mg of dobutamine. To stabilize this solution it is enough to add from 10 to 100 mg of lipoic acid. The solution has a pH of from 3.5 to 4.5, preferably from 3.8 to 4.2.

The solution is preferably prepared by adding the amount of lipoic acid and glychocolic acid in about 70 % of the final volume of water. Lipoic acid is solubilized by adding a base, preferably sodium hydroxide. Then dobutamine chlorohydrate is added under stirring. The pH is maintained in the range from 3.5 and 4.5, preferably from 3.8 to 4.2. The solution is brought to the final volume with water.

Another preferred embodiment of the invention concerns the preparation of aqueous solutions of diclofenac stabilized by lipoic acid. Since diclofenac has low solubility in water, it is necessary to add one or more cosolvents. Suitable cosolvents are alcohols and glycols, such as propylene glycol, ethylene glycol, benzyl alcohol, and mixtures thereof. Normally, diclofenac is commercialized in 3 ml vials containing 75 mg of diclofenac, however, if desired, the solution can be prepared at different concentrations. When preparing 3 ml vials containing 75 mg of diclofenac, the total amount of cosolvent can vary from 300 mg to 1 g. Preferred cosolvents are propylene glycol and benzyl alcohol. Benzyl alcohol is added in an amount preferably comprised between 250 mg and 900 mg and propylene glycol in an amount preferably comprised between 50 mg and 250 mg. The amount of lipoic acid is preferably comprised between 5 and 50 mg, more preferably from 5 to 25 mg.

In a preferred method of preparation of the solution, 50 % of the final amount of water is placed in a proper vessel, and then under stirring propylene glycol, benzyl alcohol, lipoic acid and diclofenac are added. The mixture is left under stirring until the ingredients are fully solubilized. The pH is controlled and maintained in the range from 8.2 to 8.8 by adding NaOH, if needed. The solution is brought to the final volume with water.

The obtained solution is then treated according to standard methods used in the pharmaceutical field to prepare sterile 3 ml vials containing 75 mg of diclofenac. For example, the solution can be filtered on a 0.22 µm filter and bottled sterilely.

In a further preferred embodiment of the invention, it is described a promethazine hydrochloride aqueous solution stabilized by lipoic acid. The solution is preferably prepared by adding lipoic acid and glychocolic acid to about 50 % of the final amount of water. NaOH is added to solubilized it. The, promethazine is added under stirring and trisodium citrate is added to buffer the pH to a value preferably comprised between 5 and 6, more preferably between 5.2 and 5.6. The solution is brought to the final volume with water. The obtained solution is then treated according to standard methods used in the pharmaceutical field to prepare sterile 2 ml vials containing 50 mg of promethazine hydrochloride. For example, the solution can be filtered on a 0.22 µm filter and bottled sterilely.

### Examples

### Preparation of a paracetamol injectable solution

9 L of sterile water for injection were placed in a proper vessel. Then, 6 g of lipoic acid were added, followed by 7 g of N-methylglucamine. The suspension was left under stirring until complete dissolution of lipoic acid. The pH of the solution was about 8 ± 0.3. Glychocolic acid (13 g) was then added under stirring and the pH brought to 9.5 by addition of a 10 % solution of NaOH. When also glychocolic acid was dissolved, 100 g of paracetamol was added and the suspension kept under stirring until complete dissolution of paracetamol. The pH was measured and brought to 6 by addition of a 3 % solution of phosphoric acid. The solution was brought to the final volume of 10 L by addition of sterile water for injection. The solution was then filtered in a sterile room by using a 0.22 micron filter, and bottled in 100 ml vials.

Vials obtained by this procedure were stored at 25°C for 1 year and at 40 °C for 6 months. The samples were analyzed by HPLC according to European Pharmacopoeia 6.0 and the amount of impurities measured was within the limits indicated in the European Pharmacopoeia 6.0.

### Preparation of a diclofenac injectable solution

In a proper vessel it was placed 150 ml of sterile water for injection. Then, under stirring, it was added 60 g of propylene glycol, 10.5 g of benzyl alcohol, 1 g of lipoic acid and 7.5 g of sodium diclofenac. The suspension was left under stirring until all ingredients were dissolved. The pH was controlled and brought to the value 8.5 ± 0.3 by addition of NaOH 10 % solution. The solution was brought to the final volume of 300 mL by addition of sterile water for injection. The obtained solution is then treated according to standard methods used in the pharmaceutical field to prepare sterile 3 ml vials containing 75 mg of diclofenac. For example, the solution can be filtered on a 0.22 µm filter and bottled sterilely.

### Preparation of a dobutamine chlorohydrate injectable solution

In a proper vessel were added 8.5 L of sterile water for injection. Then, under stirring, 1.25 g of lipoic acid and 0.6 g of glychocolic acid were added and lipoic acid was solubilized by addition of NaOH. 140.1 g of dobutamine chlorohydrate were added under stirring and the pH brought to 4 ± 0.2 by adding HCl or NaOH. Then, the solution is brought to the final volume of 10 L by adding sterile water for injection. The obtained solution is then treated according to standard methods used in the pharmaceutical field to prepare sterile 20 ml vials containing 250 mg of dobutamine. For example, the solution can be filtered on a 0.22 µm filter and bottled sterilely.

### Preparation of a promethazine hydrochloride injectable solution

In a proper vessel were added 8 L of sterile water for injection. Then, under stirring, 15 g of lipoic acid and 8 g of glychocolic acid were added and lipoic acid was solubilized by addition of NaOH. 250 g of promethazine hydrochloride were added under stirring and the pH brought to 5.4 ± 0.2 by adding citric acid or NaOH. Then, the solution was brought to the final volume of 10 L by adding sterile water for injection. The obtained solution was then treated according to standard methods used in the pharmaceutical field to prepare sterile 2 ml vials containing 50 mg of promethazine hydrochloride. For example, the solution can be filtered on a 0.22 µm filter and bottled sterilely.

## Claims

1. Aqueous pharmaceutical solution **characterized in that** it comprises a pharmaceutical compound and lipoic acid as an antioxidant.

2. Aqueous solution according to claim 1 further comprising a non-ionic surfactant in a weight ratio with lipoic acid comprised between 10:1 and 1:10

3. Aqueous solution according to claim 2 wherein the pharmaceutical compound is paracetamol.

4. Paracetamol solution according to claim 3, further comprising an aminosugar.

5. Paracetamol solution according to claims 3-4, wherein the paracetamol is present in a concentration comprised between 5 and 20 g/L.

6. Paracetamol solution according to claims 4-5, wherein the amino sugar is N-methylglucamine and it is present in weight ratio with paracetamol comprised between 0.01:1 and 0.5:1.

7. Paracetamol solution according to claims 3-6, wherein the non-ionic surfactant is glychocolic acid.

8. Paracetamol solution according to claims 3-7, wherein the concentration of lipoic acid is comprised between 0.05 g/L and 2 g/L.

9. Paracetamol solution according to claims 3-8 wherein the solution is not degassed to remove oxygen.

10. Aqueous solution according to claims 1-2 wherein the pharmaceutical compound is fructose 1,6 diphosphate.

11. Aqueous solution according to claims 1-2 wherein the pharmaceutical compound is diclofenac.

12. Aqueous solution according to claims 1-2 wherein the pharmaceutical compound is dobutamine chlorohydrate.

13. Aqueous solution according to claims 1-2 wherein the pharmaceutical compound is promethazine hydrochloride.

14. Process for the preparation of a paracetamol aqueous solution according to claims 3-9 comprising:
a. dissolving lipoic acid and aminosugar in water;
b. adding a non-ionic surfactant
c. correcting the pH to a value in the range from 9 to 10 by adding a base;
d. adding paracetamol and keeping the dispersion under stirring until complete solubilization is obtained;
e. correcting the pH is brought to a value close to neutral, preferably from 5 to 8, more preferably from 5.5 to 6.5, most preferably about 6, by adding an acid, preferably phosphoric acid; the solution is brought to the final volume by addition of water and the pH of the final solution controlled and corrected to the previous value by adding the acid or the base previously used.
